(19) Europäisches Patentamt
European Patent Office
Office européen des brevets

(11) Publication number: **0 162 333**
**B1**

# (12) EUROPEAN PATENT SPECIFICATION

(45) Date of publication of patent specification: **27.06.90**

(21) Application number: **85105110.2**

(22) Date of filing: **26.04.85**

(51) Int. Cl.$^5$: **C 07 D 513/06,**
C 07 D 513/16, A 61 K 31/54
// (C07D513/06, 279:00, 221:00),(C07D513/16, 317:00, 279:00, 221:00)

## (54) Quino-benzothiazine antibacterial compounds.

(30) Priority: **26.04.84 US 604190**
**26.04.84 US 604399**
**26.04.84 US 604338**

(43) Date of publication of application:
**27.11.85 Bulletin 85/48**

(45) Publication of the grant of the patent:
**27.06.90 Bulletin 90/26**

(84) Designated Contracting States:
**BE CH DE FR GB IT LI NL SE**

(56) References cited:
**EP-A-0 058 392**
**GB-A-2 010 817**

(73) Proprietor: **ABBOTT LABORATORIES**
**14th Street and Sheridan Road North St**
**North Chicago Illinois 60064 (US)**

(72) Inventor: **Chu, Daniel Tim-Wo**
**204 Ashland Court**
**Vernon Hills, IL 60061 (US)**

(74) Representative: **Modiano, Guido et al**
**MODIANO, JOSIF, PISANTY & STAUB Modiano & Associati Via Meravigli, 16**
**I-20123 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 162 333 B1

**Description**

This invention relates to new quino-benzothiazine derivatives having antibacterial properties, compositions containing the new quino-benzothiazone derivatives and methods of treating mammalian patients with the new quino-benzothiazine derivatives.

It is known that certain quinoline compounds exhibit antibacterial properties, notably certain 7-piperazinyl-4-oxo-1,4-dihydroquinoline-3-carboxylic acid derivatives which are substituted in the 1 position with an alkyl, benzyl or acetyl substituent. U.S. patent No. 4,292,317 discloses derivatives of 7-piperazinyl-4-oxo-1,4-dihydroquinoline-3-carboxylic acids wherein the 1 position is substituted by an alkyl group or a vinyl group. In U.S. Patent No. 4,284,629 there are disclosed various 4-oxo-1,4-dihydroquinoline-3-carboxylic acids in which the 1 position is substituted with a cycloalkyl group.

This invention relates to novel antibacterial agents having the formula:

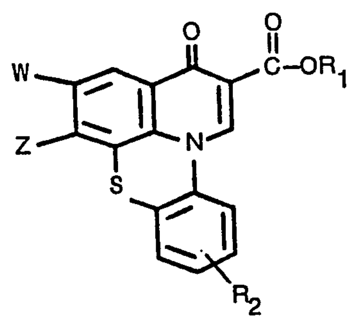

(I)

wherein $R_2$ is one or more of hydrogen, halogen, $C_1$ to $C_6$ alkyl including substituted derivatives thereof, methylenedioxy, a group having the formula —O—$R_3$ wherein $R_3$ is hydrogen or $C_1$ to $C_6$ alkyl and an amine having the formula:

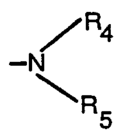

wherein

$R_4$ and $R_5$ are each independently hydrogen or $C_1$ to $C_6$ alkyl, W is halogen or hydrogen.

$R_1$ is hydrogen or a carboxy-protecting group.

Z is an amino group having the formula:

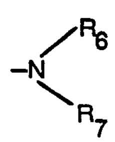

wherein $R_6$ is hydrogen or $C_1$ to $C_{10}$ alkyl as well as the corresponding halo and hydroxy substituted derivatives thereof, and $R_7$ is alkyl or substituted alkyl, as described above with reference to $R_6$, or an amino group, a mono-($C_1$ to $C_6$) alkylamino group or a di-($C_1$ to $C_6$)alkylamino group.

Alternatively, Z can be a pyridine ring substituted or unsubstituted, suitable substituents including $C_1$ to $C_6$ alkyl, halogen, a group of the formula —Y—$R_{12}$ wherein Y is —O— or —S— and $R_{12}$ is lower alkyl hydroxy, alkanoyl containing 1 to 6 carbon atoms, alkanoylamido containing 1 to 6 carbon atoms and an amine of the formula:

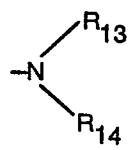

wherein $R_{13}$ and $R_{14}$ are each independently selected from the group consisting of hydrogen, $C_1$ to $C_6$ alkyl and substituted derivatives thereof.

Alternatively, Z can be an aliphatic heterocyclic ring containing 4 to 7 atoms, and preferably 5 to 6 atoms as well as substituted derivatives thereof.

W is halogen or hydrogen or, alternatively, W and Z together can form a methylenedioxy bridge.

As used herein, the term "halogen" refers to chloro, bromo, fluoro and iodo groups, while the term "$C_1$ to $C_6$ alkyl" refers to lower alkyl groups including methyl, ethyl, propyl, isopropyl and butyl.

As used herein, the term $C_1$ to $C_6$ alkyl and substituted derivatives thereof refers to hydroxy and halo-substituted derivatives of $C_1$ to $C_6$ alkyl. Such groups include, for example, a chloromethyl group, a chloroethyl group, a chloropropyl group, a hydroxyethyl group, and a trifluoromethyl group.

$R_2$ can also be a group of the formula —Y—$R_3$. Representative groups of this type include a hydroxy group, a mercapto group, a lower alkoxy group, such as methoxy, ethoxy, propoxy, as well as the thio analogs thereof, namely a methylmercapto group, and an ethylmercapto group.

As used herein, the term "carboxy-protecting group" refers to and includes the residue of a carboxylic acid ester group. Such carboxy-protecting groups are well known to those skilled in the art, having been extensively used in the protection of carboxyl groups in the penicillin and cephalosporin fields, as described in U.S. Patent Nos. 3,840,556 and 3,719,667, the disclosures of which are incorporated herein by reference. In general, such carboxy-protecting groups can be relatively easily cleaved to yield the corresponding free carboxy group. Representative protecting groups include $C_1$—$C_8$ alkyl (e.g., methyl, ethyl, tertiary butyl), substituted alkyl (e.g., dimethylaminoethyl), benzyl and substituted derivatives thereof such as alkoxy and nitrobenzyl groups; also suitable are acyl groups such as pivaloyloxymethyl groups.

The aliphatic heterocyclic rings representing Z are, in accordance with the preferred practice of the invention, aliphatic heterocyclic rings containing 1 or 2 hetero atoms which are selected from the group consisting of S, O and N, with the remaining atoms in the aliphatic heterocyclic ring being carbon atoms, as well as substituted derivatives thereof. In accordance with the practice of the invention, the aliphatic heterocyclic ring has the formula:

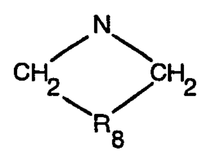

wherein $R_8$ is selected from the group consisting of dimethylene and a group of the formula —$CH_2$—$R_9$—$CH_2$ wherein $R_9$ is selected from the group consisting of —S—, —O—, —N— and —$CH_2$—. Also included are substituted derivatives of such heterocyclic rings wherein said substituents are selected from the group consisting of a $C_1$ to $C_6$ alkyl group; an amino group having the formula:

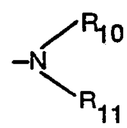

wherein $R_{10}$ and $R_{11}$ are each independently selected from the group consisting of hydrogen, $C_1$ to $C_6$ alkyl and hydroxy and halo substituted derivatives thereof; $C_1$ to $C_6$ hydroxyalkyl; hydroxy; alkanoyl containing 1 to 6 carbon atoms; and alkanoylamido containing 1 to 6 carbon atoms.

Illustrative of such heterocyclic groups are azetidinyl groups, piperazinyl groups, 4-acylpiperazinyl groups, piperidinyl groups, pyrrolidinyl groups, morpholino groups, thiomorpholino groups and homopiperazinyl groups (i.e., hexahydro-1-H-1,4-diazepinyl).

Also included within the scope of the present invention are pharmaceutically acceptable salts of the foregoing compounds. As used herein, the term "pharmaceutically acceptable salts" refers to nontoxic acid addition salts and alkaline earth metal salts of the compounds of Formula IA. The salts can be prepared *in situ* during the final isolation and purification of the compounds of Formula IA, or separately by reacting the free base or acid functions with a suitable organic acid or base. Representative acid addition salts include the hydrochloride, hydrobromide, sulphate, bisulphate, acetate, oxalate, valerate, oleate, palmitate, stearate, laurate, borate, benzoate, lactate, phosphate, tosylate, mesylate, citrate, maleate, fumarate, succinate, tartrate, glucoheptonate, lactobionate, lauryl sulfate salts and the like.

Representative alkali or alkaline earth metal salts include the sodium, calcium, potassium and magnesium salts. It has been found that the compounds of the present invention possess antibacterial activity against a wide spectrum of gram positive and gram negative bacteria, as well as enterobacteria. The compounds of the invention are therefore useful in the antibiotic treatment of susceptible bacterial infections in both humans and animals. In addition, the compounds, by reason of their *in vitro* activity, may be used in scrub solutions for surface inhibition of bacterial growth.

Susceptible organisms generally include those gram positive and gram negative, aerobic and anaerobic organisms whose growth can be inhibited by the compounds of the invention such as *Staphylococcus, Lactobacillus, Streptococcus, Sarcina, Escherichia, Enterobacter, Klebsiella, Pseudomonas, Acinetobacter, Proteus, Citrobacter, Nisseria, Baccillus, Bacteroides, Peptococcus, Clostridium, Salmonella, Shigella, Serratia, Haemophilus, Brucella*, and other organisms. In addition to exhibiting highly effective antibacterial activity, the compounds of the invention exhibit increased and

improved solubility characteristics as compared with prior quinoline-3-carboxylic acid compounds in the art.

The compounds of Formula IA may also be formulated into compositions together with pharmaceutically acceptable carriers for parenteral injection, for oral administration in solid or liquid form, for rectal administration, and the like.

Compositions according to the invention for parenteral injection may comprise pharmaceutically acceptable sterile aqueous or nonaqueous solutions, suspensions or emulsions. Examples of suitable nonaqueous carriers, diluents, solvents or vehicles include propylene glycol, polyethylene glycol, vegetable oils, such as olive oil, and injectable organic esters such as ethyl oleate. Such compositions may also contain adjuvants such as preserving, wetting, emulsifying, and dispersing agents. They may be sterilized, for example, by filtration through a bacteria-retaining filter, or by incorporating sterilizing agents into the compositions. They can also be manufactured in the form of sterile solid compositions which can be dissolved in sterile water, or some other sterile injectable medium immediately before use.

Solid dosage forms for oral administration include capsules, tablets, pills, powders and granules. In such solid dosage forms, the active compound is admixed with at least one inert diluent such as sucrose, lactose or starch. Such dosage forms can also comprise, as is normal practice, additional substances other than diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups and elixers containing inert diluents commonly used in the art, such as water. Besides such inert diluents, compositions can also include adjuvants, such as wetting agents, emulsifying and suspending agents, and sweetening, flavoring and perfuming agents.

Compositions for rectal administration are preferably suppositories which may contain, in addition to the active substance, excipients such as cocoa butter or a suppository wax.

Actual dosage levels of active ingredient in the compositions of the invention may be varied so as to obtain an amount of active ingredient effective to achieve antibacterial activity in accordance with the desired method of administration. The selected dosage level therefore depends upon the nature of the active compound administered, the route of administration, the desired duration of treatment and other factors. Generally, daily dosage levels of the compounds of Formula I of about 0.1 to about 750, more preferably about 0.25 to about 500 and most preferably about 0.5 to about 300 mg. of active ingredient per kg. of body weight are effective when administered orally to a mammalian patient suffering from an infection caused by a susceptible organism. If desired, the daily dose may be divided into multiple doses for administration, e.g., two to four times per day.

Compounds according to the invention wherein W is F can be prepared by the reaction illustrated below:

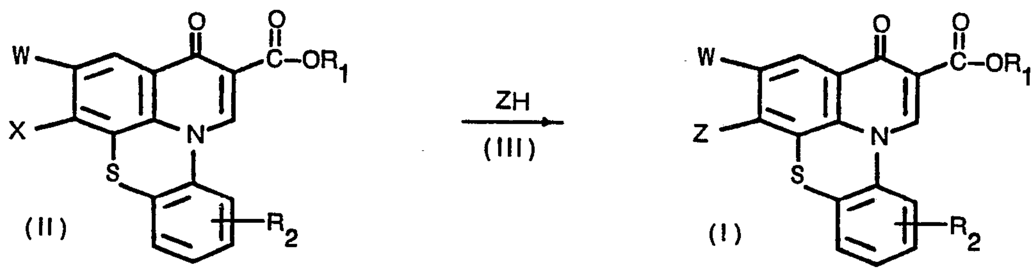

wherein X is a halogen and $R_1$, $R_2$, W and Z are the same as described above with the exception that Z is not pyridine.

The reaction may be performed by heating a compound of the formula (II) with an amine of formula (III) at a temperature of from 20°C to 200°C, and preferably from 70°C to 150°C, in the presence of a suitable organic polar solvent such as dimethylsulfoxide, sulfolane, dimethylformamide, dimethylacetamide, 1-methyl-2-pyrrolidinone or water. It is desirable to carry out the reaction in the presence of an acid-acceptor such as triethylamine, potassium carbonate and the like at a molar ratio of 1.0 to 1.2 mole of the acid-acceptor per mole of the compound of the formula (II). The amine (III) can also be used as acid acceptor in which 2 or more molar excess of this reagent is used. The compounds of the formula (II) may be prepared in accordance with the following reaction scheme in which R is as described above and X can be independently identical or non-identical halogen.

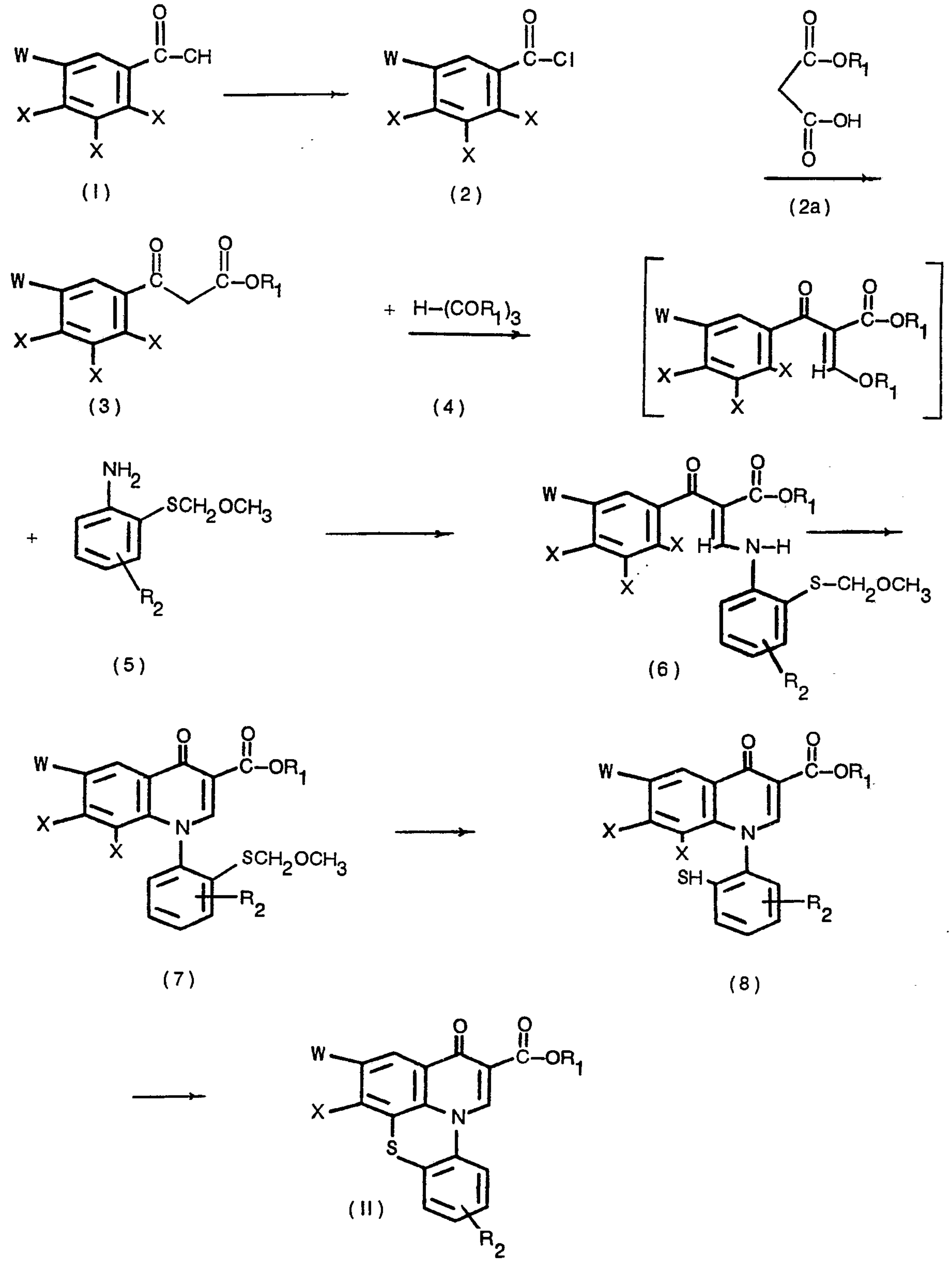
(1) (2) (2a) (3) (4) (5) (6) (7) (8) (II)

In accordance with the foregoing reaction scheme, the 2,3,4-trihalo-5-flurobenzonic acid (1) is heated with thionyl chloride to produce the corresponding acid chloride (2). Displacement of the acid chloride (2) with malonic acid half ester (2a) in the presence of n-butyl lithium yields the β-ketoester (3).

The β-ketoester (3) is then treated with a trialkylorthoformate (4) in the presence of an acid anhydride, preferably acetic anhydride, followed by reaction with substituted or unsubstituted o-methoxymethylthioaniline (5) to obtain the enaminoketoester (6). In the trialkylortho formate (4), $R_4$ may be an alkyl group of, for example, from 1 to 10 carbon atoms, but is preferably loweralkyl, such as ethyl. Reaction with the trialkylorthoformate is preferably conducted at elevated temperatures, such as from

about 50°C to about 150°C, preferably from about 100°C to about 140°C, to obtain an oily liquid, which may be isolated or unisolated, as desired (shown in brackets in the reaction scheme). Reaction of the latter with the substituted or unsubstituted o-methoxymethylthioaniline (5) is preferably conducted in an appropriate aprotic or nonaprotic solvent, preferably methylene chloride or tetrahydrofuran, and may be conducted at room or suitable elevated temperature, as desired.

The enaminoketoester (6) is then cyclized, such as by treatment with a strong base as defined above, preferably sodium hydride, to obtain the 1,4-dihydro-4-oxo-quinoline-3-carboxylic acid ester (7). Cyclization is conducted in the presence of an aprotic solvent, such as dimethoxyethane, bis(2-methoxyethyl)ether, dimethylformamide, tetrahydrofuran or chlorobenzene, and is preferably conducted at temperatures of about 20°C to about 145°C, more preferably at the reflux temperature of the solvent employed.

The methoxymethylthio ether moiety of (1) is then removed by strong mineral acid or by boron trichloride treatment to give the thiophenol compound (8). Cyclization of (8) with a strong base as defined above, preferably sodium hydride, yields the 1-halo-2-fluoro-4-oxo-4H-quino[2,3,4-i,j][1,4]benzothiazine-5-carboxylic acid ester (II), ($R_1$ = alkyl). Cyclization is conducted in the presence of an aprotic solvent as defined above.

The ester (II) is subjected to hydrolysis, such as by treatment with sodium hydroxide, or dilute mineral acid to form the free acid (II) ($R_1 = H$).

The 4-oxo-4H-quino[2,3,4-i,j][1,4]benzothiazine-5-carboxylic acid (I, $R_1 = H$) can then be converted into the corresponding ester (I, $R_1 = H$), if desired, by conventional esterification procedures, such as by treating the free acid (I, $R_1 = H$) with the appropriate alcohol in the presence of an acid catalyst, by converting the free acid (I, $R_1 = H$) into the corresponding acid chloride followed by displacement of the chloro radical with the appropriate alcohol, or by treating the sodium salt of the acid (I, $R_1 = H$) with a suitable reactive halide, such as chloromethylpivalate or dimethylaminoethylchloride in dimethoxyethane to obtain, for example, the pivaloyloxymethyl ester (I) wherein $R_1$ is $-CH_2OCOC(CH_3)_3$, or dimethylaminoethylester (I) wherein $R_1$ is $-CH_2CH_2N(CH_3)_2$.

The foregoing may be better understood from the following examples, which are presented for purposes of illustration and are not intended to limit the scope of the inventive concepts. As used in the following examples, the references to compounds, such as (1), (2), (3), etc., and to substituents, such as $R_1$, $R_2$, etc., refer to the corresponding compounds and substituents in the foregoing reaction scheme and in formulae I, II and III.

To a cold solution of methoxymethylthio ether (7) (4 g.) in 100 ml. methylene chloride is added 10 ml. of boron trichloride with stirring. After 15 minutes at 0°C, the solution is evaporated to dryness under reduced pressure at room temperature, yielding the crude thiophenol (8). This is then dissolved in 50 ml. O-dichlorobenzene. 400 mg. of sodium hydride is added and the mixture is heated at 135°C for 24 hours. It is cooled and 300 ml. water is added and the mixture is extracted with methylene chloride (3 × 300 ml.). It is dried and evaporated to dryness. Purification by chromatography on silica gel column yields (II), ($R_1 = C_2H_5$, $X = F$, $R_2 = H$).

(d) To a suspension of 7 g. of (II) ($R_1 = C_2H_5$, $X = F$, $R_2 = H$) in 30 ml. THF is added a sodium hydroxide solution (0.8 g. in 20 ml. water). The mixture is heated under nitrogen at 80°C for 1 hour resulting in a clear solution which is evaporated under reduced pressure to dryness. The solid is dissolved in 200 ml. water and 2.5 ml. acetic acid is added. The resulting precipitate is filtered and washed with cold water, crystallized from dimethylformamide (DMF) to produce 1,2-difluoro-4-oxo-4H-quino[2,3,4-i,j][1,4]benzothiazine-5-carboxylic acid (II) ($R_1 = H$, $X = F$, $R_2 = H$).

(e) To a solution of 3.5 g. of 1,2-difluoro-4-oxo-4H-quino[2,3,4-i,j][1,4]benzothiazine-5-carboxylic acid (II) ($R_1 = H$, $X = F$, $R_2 = H$) in 25 ml. of 1-methyl-2-pyrrolidinone at 115°C is added 4 ml. piperazine. After stirring at 100°C for 20 hours, the solvent is removed by reduced pressure to dryness. Ethanol is added to the residue and the resulting mixture is filtered and washed with ether and then washed with very small amounts of cold water to give (I)

($R_1$=H, $R_2$=H, Z= 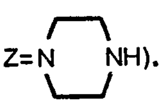 ).

The resulting dried solid (I) is suspended in 30 ml. water and 11 ml. 1N HCl is added to and warmed to dissolve. Removal of the solvent under reduced pressure gives hydrochloride salt of 1-(1-piperazinyl)-2-fluoro-4-oxo-4H-quino[2,3,4-i,j][1,4]benzothiazine-5-carboxylic acid (I)

($R_1$=H, $R_2$=H, Z= 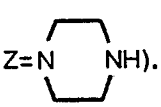 ).

Example 1

1-(1-piperazinyl)-2-fluoro-4-oxo-4H-quino[2,3,4-i,j][1,4]benzothiazine-5-carboxylic acid

(a) To a solution of 3.9 g. of 2,3,4,5-tetrafluorobenzoic acid in 20 ml. of methylene chloride are added

4 ml. of thionylchloride. After refluxing for 30 minutes, the reaction mixture is evaporated to dryness to give 4.95 g. of the acid chloride.

This is slowly added to a dry ice-cooled solution of 3.5 g. of malonic acid monoethyl ester in 35 ml. of tetrahydrofuran, 37.9 ml. of 1.4 M. n-butyl lithium in THF, and the flask is warmed to −5°C for 5 minutes and then is cooled back to −70°C. To the resulting solution there is added 4.95 g. of the acid chloride in 10 ml. of THF. The cooling bath is removed, and the solution allowed to warm to room temperature after an hour. The solution is then partitioned between 1N ECl and ether. The ether portion is washed with $NaECO_3$ and dried over $MgSO_4$, and then evaporated to obtain the liquid β-ketoester (3). ($R_1$ = $C_2H_5$, X = F).

(b) A solution of 20 g. of the above 3-ketoester (3) in 18.5 ml of triethylorthoformate and 45 ml. of acetic anhydride is heated at 135°C for 1-1/2 hours with the removal of the ethyl acetate formed during the reaction. The solution is evaporated under reduced pressure to a mobile oil. The oil is then dissolved in 200 ml. of methylene chloride and 10 g. of 2-methoxymethylthioaniline is added into the solution. After 1 hour, the solution is evaporated to dryness and crystallized from 200 ml. of hexane and 5 ml. of ether yielding (6), wherein $R_1$ = $C_2H_5$, $R_2$ = H, X = F.

(c) To a cold solution of 15 g. of the preceding product (6), $R_1$ = $C_2H_5$, $R_2$ = H, X = F, in 150 ml. tetrahydrofuran (THF) is slowly added 1.4 g. of a 60% sodium hydride-in-oil suspension. The mixture is refluxed for 6 hours and is cooled and diluted with water to a volume of 1.5 liters. The mixture is then filtered to obtain (7) wherein $R_1$ = $C_2H_5$, $R_2$ = H, X = F.

To the hydrochloride salt is added with a dilute aqueous solution of sodium hydroxide to pH 7 to obtain 1-(1-piperazinyl)-2-fluoro-4-oxo-4H-quino[2,3,4-i,j][1,4]benzothiazine-5-carboxylic acid (I).

(f) Alternately, the title compound is prepared as follows: To a suspension of 5 g. of compound (II) (product of 1(d)) in 40 ml. of 1-methyl-2-pyrrolidinone at 120°C under nitrogen atmosphere is added 10 ml. of N-carboethoxy-piperazine. After 20 hours, the solvent is removed under reduced pressure, and the residue is suspended in 150 ml. ethanol and refluxed for 1/2 hour. The reaction mixture is then cooled and filtered. The resulting solid is washed with cold ethanol and water to obtain compound (I).

($R_1$=H, $R_2$=H, Z= 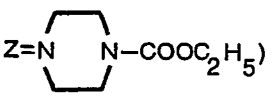

To a suspension of 5 g. of the preceding compound (I)

($R_1$=H, $R_2$=H, Z= 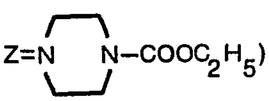

in 25 ml. of ethanol at 80°C is added 50 ml. of 10% NaOH solution. The solution is heated at 80°C for 6 hours. The solvent is removed and the solid is dissolved in 100 ml. water. The pH of the solution is adjusted to pH 7 by the addition of 10% acetic acid. The precipitate is filtered and washed with cold water yielding 1-(1-piperazinyl)-2-fluoro-4-oxo-4H-quino[2,3,4-i,j][1,4]benzothiazine-5-carboxylic acid (I)

($R_1$=H, $R_2$=H, Z= 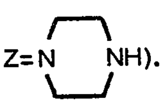

Example 2

1-[1-(4-methyl)piperazinyl]-2-fluoro-4-oxo-4H-quino[2,3,4-i,j][1,4]benzothiazine-5-carboxylic acid

The procedure of Example 1 is repeated replacing piperazine in Example 1(e) with N-methylpiperazine to obtain 1-[1-(4-methyl)piperazinyl]-2-fluoro-4-oxo-4H-quino[2,3,4-i,j][1,4]benzothiazine-5-carboxylic acid (I)

($R_1$=H, $R_2$=H, Z= 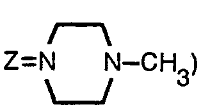

and its hydrochloride salt.

Example 3

1-(1-pyrrolidinyl)-2-fluoro-4-oxo-4H-quino[2,3,4-i,j][1,4]benzothiazine-5-carboxylic acid

In the described fashion as Example 1, replacing piperazine in Example 1(e) with pyrrolidine, one can obtain 1-(1-pyrrolidinyl)-2-fluoro-4-oxo-4H-quino[2,3,4-i,j][1,4]benzothiazine-5-carboxylic acid (I)

($R_1$=$R_2$=H, Z = 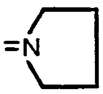 ).

Example 4

1-(1-3-hydroxypyrrolidinyl)-2-fluoro-4-oxo-4H-quino[2,3,4-i,j][1,4]benzothiazine-5-carboxylic acid

The procedure of Example 1 can be repeated replacing piperazine in Example 1(e) with 3-hydroxypyrrolidine to obtain 1-(1-3-hydroxypyrrolidinyl)-2-fluoro-4-oxo-4H-quino[2,3,4-i,j][1,4]benzothiazine-5-carboxylic acid (I)

($R_1$=$R_1$=H, Z= 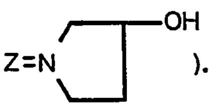 ).

Example 5

1-(1-3-aminopyrrolidinyl)-2-fluoro-4-oxo-4H-quino[2,3,4-i,j][1,4]benzothiazine-5-carboxylic acid

(a) In the described fashion as Example 1 replacing piperazine in Example 1(e) with 3-acetamidopyrrolidine, one can obtain 1-(1-3-acetamidopyrrolidinyl)-2-fluoro-4-oxo-4H-quino[2,3,4-i,j][1,4]benzothiazine-5-carboxylic acid (I)

($R_1$=$R_2$=H, Z= 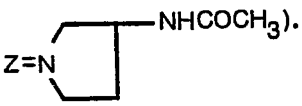).

(b) The product of the above reaction can then be hydrolyzed with hydrochloric acid at 80°C to give the hydrochloride salt of 1-(1-3-aminopyrrolidinyl)-2-fluoro-4-oxo-4H-quino[2,3,4-i,j][1,4]benzothiazine-5-carboxylic acid (I)

($R_1$=$R_2$=H, Z= 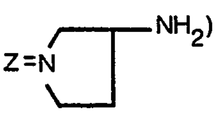)

and its hydro-chloride salt.

Example 6

1-(1-3-methylaminopyrrolidinyl)-2-fluoro-4-oxo-4H-quino[2,3,4-i,j][1,4]benzothiazine-5-carboxylic acid

In the described fashion as Example 1 replacing piperazine in Example 1(e) with 3-N-formyl-N-methyl-pyrrolidine, one can obtain 1-(1-3-N-formyl N-methylaminopyrrolidinyl)-2-fluoro-4-oxo-4H-quino[2,3,4-i,j][1,4]benzothiazine-5-carboxylic acid (I)

($R_1$=$R_2$=H, Z= 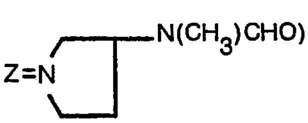)

(B) The product of the above reaction can then be hydrolyzed with hydrochloric acid at 80°C to give 1-(1-3-methylaminopyrrolidinyl)-2-fluoro-4-oxo-4H-quino[2,3,4-i,j][1,4]benzothiazine-5-carboxylic acid (I),

($R_1$=$R_2$=H, Z= 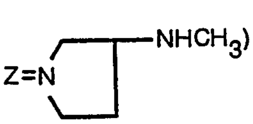)

and its hydrochloride salt.

Example 7

1-(1-3-dimethylaminopyrrolidinyl)-2-fluoro-4-oxo-4H-quino[2,3,4-i,j][1,4]benzothiazine-5-carboxylic acid

The procedure of Example 1 is repeated replacing piperazine in Example 1(e) with 3-dimethylaminopyrrolidine to obtain 1-(1-3-dimethylaminopyrrolidinyl)-2-fluoro-4-oxo-4H-quino[2,3,4-i,j][1,4]benzothiazine-5-carboxylic acid (I) and its hydrochloride salt

($R_1$=$R_2$=H, Z= 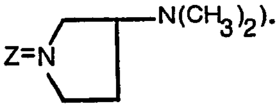 ).

Example 8

1-(1-piperidinyl)-2-fluoro-4-oxo-4H-quino[2,3,4-i,j][1,4]benzothiazine-5-carboxylic acid

The procedure of Example 1 can be repeated replacing piperazine in Example 1(e) with piperidine to obtain 1-(1-piperadinyl)-2-fluoro-4-oxo-4H-quino[2,3,4-i,j][1,4]benzothiazine-5-carboxylic acid (I)

($R_1$=$R_2$=H, Z= 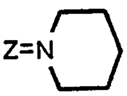 ).

Example 9

1-(4-morpholinyl)-2-fluoro-4-oxo-4H-quino[2,3,4-i,j][1,4]benzothiazine-5-carboxylic acid

In the described fashion as Example 1, replacing piperazine in Example 1(e) with morpholine, one can obtain 1-(4-morpholinyl)-2-fluoro-4-oxo-4H-quino[2,3,4-i,j][1,4]benzothiazine-5-carboxylic acid (I)

($R_1$=$R_2$=H, Z= 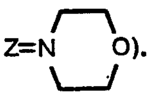 ).

Example 10

1-(4-thiomorpholinyl)-2-fluoro-4-oxo-4H-quino[2,3,4-i,j][1,4]benzothiazine-5-carboxylic acid

The procedure of Example 1 is repeated replacing piperazine in Example 1(e) with thiomorpholine to obtain 1-(4-thiomorpholinyl)-2-fluoro-4-oxo-4H-quino[2,3,4-i,j][1,4]benzothiazine-5-carboxylic acid (I)

($R_1$=$R_2$=H, Z= 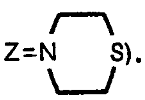 ).

Example 11

1-(1-3,5-dimethylpiperazinyl)-2-fluoro-4-oxo-4H-quino[2,3,4-i,j][1,4]benzothiazine-5-carboxylic acid

In the described fashion as Example 1, replacing piperazine in Example 1(e) with 2,6-dimethylpiperazine, one obtains 1-(1-3,5-dimethylpiperazinyl)-2-fluoro-4-oxo-4H-quino[2,3,4-i,j][1,4]benzothiazine-5-carboxylic acid (I) and its hydrochloride salt.

($R_1$=$R_2$=H, Z= 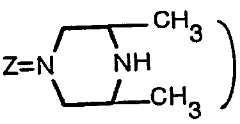 ).

Example 12

1-(1-homopiperazinyl)-2-fluoro-4-oxo-4H-quino[2,3,4-i,j][1,4]benzothiazine-5-carboxylic acid

The procedure of Example 1 can be repeated replacing piperazine in Example 1(e) with homopiperazine to obtain 1-(1-homopiperazinyl)-2-fluoro-4-oxo-4H-quino[2,3,4-i,j][1,4]benzothiazine-5-carboxylic acid (I) and its hydrochloride salt.

($R_1$=$R_2$=H, Z= 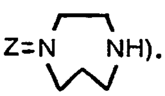 ).

Example 13

1-dimethylamino-2-fluoro-4-oxo-4H-quino[2,3,4-i,j][1,4]benzothiazine-5-carboxylic acid

In the described fashion as Example 1, replacing piperazine in Example 1(e) with dimethylamine, one can obtain 1-dimethylamino-2-fluoro-4-oxo-4H-quino[2,3,4-i,j][1,4]benzothiazine-5-carboxylic acid (I) ($R_1$ = $R_2$ = H, Z = $N(CH_3)_2$).

Example 14

1-(N-2-hydroxyethylamino)-2-fluoro-4-oxo-4H-quino[2,3,4-i,j][1,4]benzothiazine-5-carboxylic acid

The procedure of Example 1 can be repeated, replacing piperazine in Example 1(e) with N-2-hydroxyethylamine to obtain 1-(N-2-hydroxyethylamino)-2-fluoro-4-oxo-4H-quino[2,3,4-i,j][1,4]benzothiazine-5-carboxylic acid (I) ($R_1$ = $R_2$ = H, Z = $NHC_2H_4$—OH).

Example 15

1-hydrazyl-2-fluoro-4-oxo-4H-quino[2,3,4-i,j][1,4]benzothiazine-5-carboxylic acid

In the described fashion as Example 1, replacing piperazine in Example 1(e) with hydrazine, one can obtain 1-hydrazyl-2-fluoro-4-oxo-4H-quino[2,3,4-i,j][1,4]benzothiazine-5-carboxylic acid ($R_1$ = $R_2$ = H, Z = $NHNH_2$).

Example 16

1-(1-piperazinyl)-2,10-difluoro-4-oxo-4H-quino[2,3,4-i,j][1,4]benzothiazine-5-carboxylic acid

(a) In the described fashion as Example 1(b), replacing 2-methoxymethylthioaniline with 2-methoxymethylthio-4-fluoroaniline, one can obtain the enaminoketoester (6) ($R_1$ = $C_2H_5$, $R_2$ = fluoro, X = F).

(b) By following the Example 1(c) and 1(d), the preceding compound (6) can yield 1,2-10-trifluoro-4-oxo-4H-quino[2,3,4-i,j][1,4]benzothiazine-5-carboxylic acid (II) ($R_1$ = H, X = F, $R_2$ = fluoro).

(c) In the described fashion as Example 1(e), the above acid (II) can give the desired 1-(1-piperazinyl)-2,10-difluoro-4-oxo-4H-quino[2,3,4-i,j][1,4]benzothiazine-5-carboxylic acid (I) and its hydrochloride salt

($R_1$=H, $R_2$=10–fluoro, 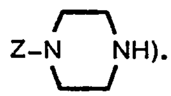).

Example 17

In the described fashion as Example 1(e), replacing the acid (II) ($R_1$ = $R_2$ = H, X = F) with the acid (II) of the product of Example 1(b) ($R_1$ = H, $R_2$ = 10-fluoro, X = F) and also replacing piperazine with an appropriate amine such as N-methylpiperazine, pyrrolidine, 3-hydroxypyrrolidine, 3-acetaminopyrrolidine, 3-N-formyl-N-methylaminopyrrolidine, 3-dimethylaminopyrrolidine, thiomorpholine, 2,6-dimethylpiperazine, homopiperazine, diethylamine and 2,2-dimethylhydrazine, one can obtain the following compounds:

(a) 1-(1-4-methylpiperazinyl)-2,10-difluoro-4-oxo-4H-quino[2,3,4-i,j][1,4]benzothiazine-5-carboxylic acid (I) and its hydrochloride salt ($R_1$ = H, $R_2$ = 10-fluoro,

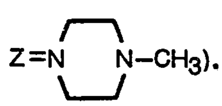).

(b) 1-(1-pyrrolidinyl)-2,10-difluoro-4-oxo-4H-quino[2,3,4-i,j][1,4]benzothiazine-5-carboxylic acid (I)

($R_1$=H, $R_2$=10–fluoro, 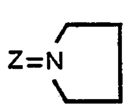).

(c) 1-(1-3-hydroxypyrrolidinyl)-2,10-difluoro-4-oxo-4H-quino[2,3,4-i,j][1,4]benzothiazine-5-carboxylic acid (I)

($R_1$=H, $R_2$=10–fluoro, 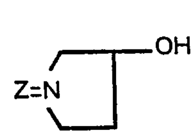).

(d) 1-(1-3-acetamidopyrrolidinyl)-2,10-difluoro-4-oxo-4H-quino[2,3,4-i,j][1,4]benzothiazine-5-carboxylic acid (I)

($R_1$=H, $R_2$=10–fluoro, Z= 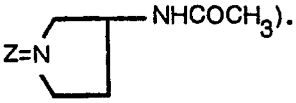 ).

(e) 1-(1-3-N-formyl-N-methylaminopyrrolidinyl)-2,10-difluoro-4-oxo-4H-quino[2,3,4-i,j][1,4]benzothiazine-5-carboxylic acid (I)

($R_1$=H, $R_2$=10–fluoro, Z= 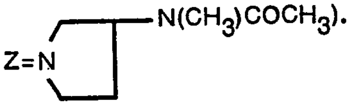 ).

(f) 1-(1-3-dimethylaminopyrrolidinyl)-2,10-difluoro-4-oxo-4H-quino[2,3,4-i,j][1,4]benzothiazine-5-carboxylic acid

($R_1$=H, $R_2$=10–fluoro, Z= 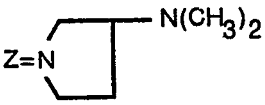 (I)

and its hydrochloride salt.

(g) 1-(1-piperidinyl)-2,10-difluoro-4-oxo-4H-quino[2,3,4-i,j][1,4]benzothiazine-5-carboxylic acid (I)

($R_1$=H, $R_2$=10–fluoro, Z= 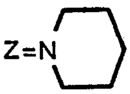 ).

(h) 1-(4-morpholinyl)-2,10-difluoro-4-oxo-4H-quino[2,3,4-i,j][1,4]benzothiazine-5-carboxylic acid (I),

($R_1$=H, $R_2$=10–fluoro, Z= 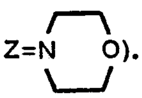 ).

(i) 1-(4-thiomorpholinyl)-2,10-difluoro-4-oxo-4H-quino[2,3,4-i,j][1,4]benzothiazine-5-carboxylic acid (I)

($R_1$=H, $R_2$=10–fluoro, Z= 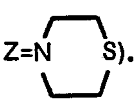 ).

(j) 1-(1-3,5-dimethylpiperazinyl)-2,10-difluoro-4-oxo-4H-quino[2,3,4-i,j][1,4]benzothiazine-5-carboxylic acid (I),

($R_1$=H, $R_2$=10–fluoro, Z= 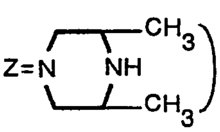 )

and its hydrochloride salt.

(k) 1-(1-homopiperazinyl)-2,10-difluoro-4-oxo-4H-quino[2,3,4-i,j][1,4]benzothiazine-5-carboxylic acid (I)

($R_1$=H, $R_2$=10–fluoro, Z= 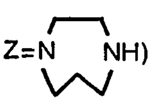 )

and its hydrochloride salt.

(l) 1-dimethylamino-2,10-difluoro-4-oxo-4H-quino[2,3,4-i,j][1,4]benzothiazine-5-carboxylic acid (I) ($R_1$ = H, $R_2$ = 10-fluoro, Z = $N(CH_3)_2$).

(m) 1-N,N-dimethylhydrazyl-2,10-difluoro-4-oxo-4H-quino[2,3,4-i,j][1,4]benzothiazine-5-carboxylic acid (I) ($R_1$ = H, $R_2$ = 10-fluoro, Z = $NHN(CH_3)_2$).

Example 18

In the described fashion of Example 5(b), the compounds of Examples 17(d) and 17(e) can give the following two compounds:

(a) 1-(1-3-aminopyrrolidinyl)-2,10-difluoro-4-oxo-4H-quino[2,3,4-i,j][1,4]benzothiazine-5-carboxylic acid (I) and its hydrochloride salt.

($R_1$=H, $R_2$=10–fluoro, Z=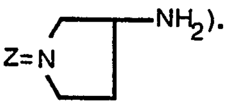

(b) 1-(1-3-methylaminopyrrolidinyl)-2,10-difluoro-4-oxo-4H-quino[2,3,4-i,j][1,4]benzothiazine-5-carboxylic acid (I) and its hydrochloride salt

($R_1$=H, $R_2$=10–fluoro, Z=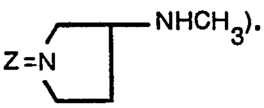

Example 19

In the described fashion as Example 1(a—d), replacing 2-methoxymethylthioaniline with an appropriate substituted 2-methoxymethylthioaniline

( 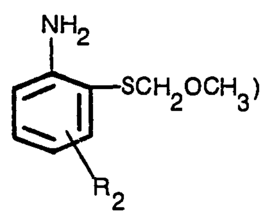

one can obtain the additional substituted 1,2-difluoro-4-oxo-4H-quino[2,3,4-i,j][1,4]benzothiazine-5-carboxylic acid (II) as listed in Table I.

TABLE I

| Substituted 2-methoxy-methylthioaniline | Compound (II) ($R_1$ = H, X = F) obtained |
|---|---|
| $R_2$ | $R_2$ |
| a) 4-methoxy | 10-methoxy |
| b) 4-methyl | 10-methyl |
| c) 4-chloro | 10-chloro |
| d) 4,6-difluoro | 8,10-difluoro |
| e) 4,5-methylenedioxy | 9,10-methylenedioxy |
| f) 4-hydroxy | 10-hydroxy |
| g) 4-dimethylamino | 10-dimethylamino |
| h) 5-fluoro | 9-fluoro |

Example 20

In the described fashion as Example 1(e), replacing the acid (II) ($R_1$ = H, $R_2$ = H, X = F) with the acid (II) of the compounds listed in Table I of Example 19 and also replacing piperazine with an appropriate amine such as N-methylpiperazine, pyrrolidine, 3-hydroxypyrrolidine, 3-acetamidopyrrolidine, 3-N-formyl-N-methylaminopyrrolidine, 3-dimethylaminopyrrolidine, piperidine, morpholine, thiomorpholine, 2,6-dimethylpiperazine, homopiperazine, dimethylamine, 2,2-dimethylhydrazine, and an extra hydrolysis step if required as in Example 5(b), one can obtain the following additional compounds as summarized in Table II.

Table II

| Piperazine replacement | Compound II used ($R_1$=H, X=F) | Compound I obtained $R_1$=H) | |
|---|---|---|---|
| ZH | $\underline{R}_2$ | $\underline{R}_2$ | $\underline{Z}$ |
| 1. piperazine | 8,10-difluoro | 8,10-difluoro | piperazinyl |
| 2. piperazine | 9,10-methylenedioxy | 9,10-methylenedioxy | piperazinyl |
| 3. piperazine | 10-hydroxy | 10-hydroxy | piperazinyl |
| 4. piperazine | 10-methyl | 10-methyl | piperazinyl |
| 5. 4-methylpiperazine | 8,10-difluoro | 8,10-difluoro | 4-methylpiperazinyl |
| 6. 4-methylpiperazine | 9,10-methylenedioxy | 9,10-methylenedioxy | 4-methylpiperazinyl |
| 7. 4-methylpiperazine | 10-hydroxy | 10-hydroxy | 4-methylpiperazinyl |
| 8. 4-methylpiperazine | 10-chloro | 10-chloro | 4-methylpiperazinyl |
| 9. 3-acetamido-pyrrolidine | 8,10-difluoro | 8,10-difluoro | 3-aminopyrrolidinyl |
| 10. 3-acetamido-pyrrolidine | 9,10-methylenedioxy | 9,10-methylenedioxy | 3-aminopyrrolidinyl |
| 11. 3-acetamido-pyrrolidine | 10-hydroxy | 10-hydroxy | 3-aminopyrrolidinyl |

Table II (continued)

| Piperazine replacement | Compound II used ($R_1$=H, Z=F) | Compound I obtained $R_1$=H) | |
|---|---|---|---|
| ZH | $R_2$ | $R_2$ | Z |
| 2. 3-acetamido-pyrrolidine | 9-fluoro | 9-fluoro | 3-aminopyrrolidinyl |
| 3. 3-methylacetamido-pyrrolidine | 8,10-difluoro | 8,10-difluoro | 3-methylaminopyrrolidinyl |
| 4. 3-methylacetamido-pyrrolidine | 9,10-methylenedioxy | 9,10-methylenedioxy | 3-methylaminopyrrolidinyl |
| 5. 3-methylacetamido-pyrrolidine | 10-hydroxy | 10-hydroxy | 3-methylaminopyrrolidinyl |
| 6. 3-methylacetamido-pyrrolidine | 10-dimethylamino | 10-dimethylamino | 3-methylaminopyrrolidinyl |
| 7. pyrrolidine | 10-methoxy | 10-methoxy | pyrrolidinyl |
| 8. 3-dimethylamino-pyrrolidine | 9,10-methylenedioxy | 9,10-methylenedioxy | 3-dimethylaminopyrrolidinyl |
| 9. 3-dimethylamino-pyrrolidine | 8,10-difluoro | 8,10-difluoro | 3-dimethylaminopyrrolidinyl |
| 10. 3-hydroxypyrrolidine | 9-fluoro | 9-fluoro | 3-hydroxypyrrolidinyl |

**Table II (continued)**

| Piperazine replacement | Compound II used ($R_1$=H, X=F) | Compound I obtained $R_1$=H) | |
|---|---|---|---|
| ZH | $R_2$ | $R_2$ | Z |
| 1. piperidine | 10-hydroxy | 10-hydroxy | piperidinyl |
| 2. morpholine | 10-chloro | 10-chloro | morpholinyl |
| 3. thiomorpholine | 10-methyl | 10-methyl | thiomorpholinyl |
| 4. N,N-dimethyl-hydrazine | 10-methoxy | 10-methoxy | N,N-dimethylhydrazinyl |
| 5. dimethylamine | 10-chloro | 10-chloro | dimethylamino |
| 6. homopiperazine hydrazine | 8,10-difluoro | 8,10-difluoro | homopiperazinyl |
| 7. 2,6-dimethyl-piperazine | 9,10-methylenedioxy | 9,10-methylenedioxy | 3,5-dimethylpiperazinyl |

The compounds of Formula IV wherein W and Z together form a methylenedioxy bridge may be prepared in accordance with the following reaction scheme in which $R_2$ is as described above and X can be independently identical or non-identical halogen.

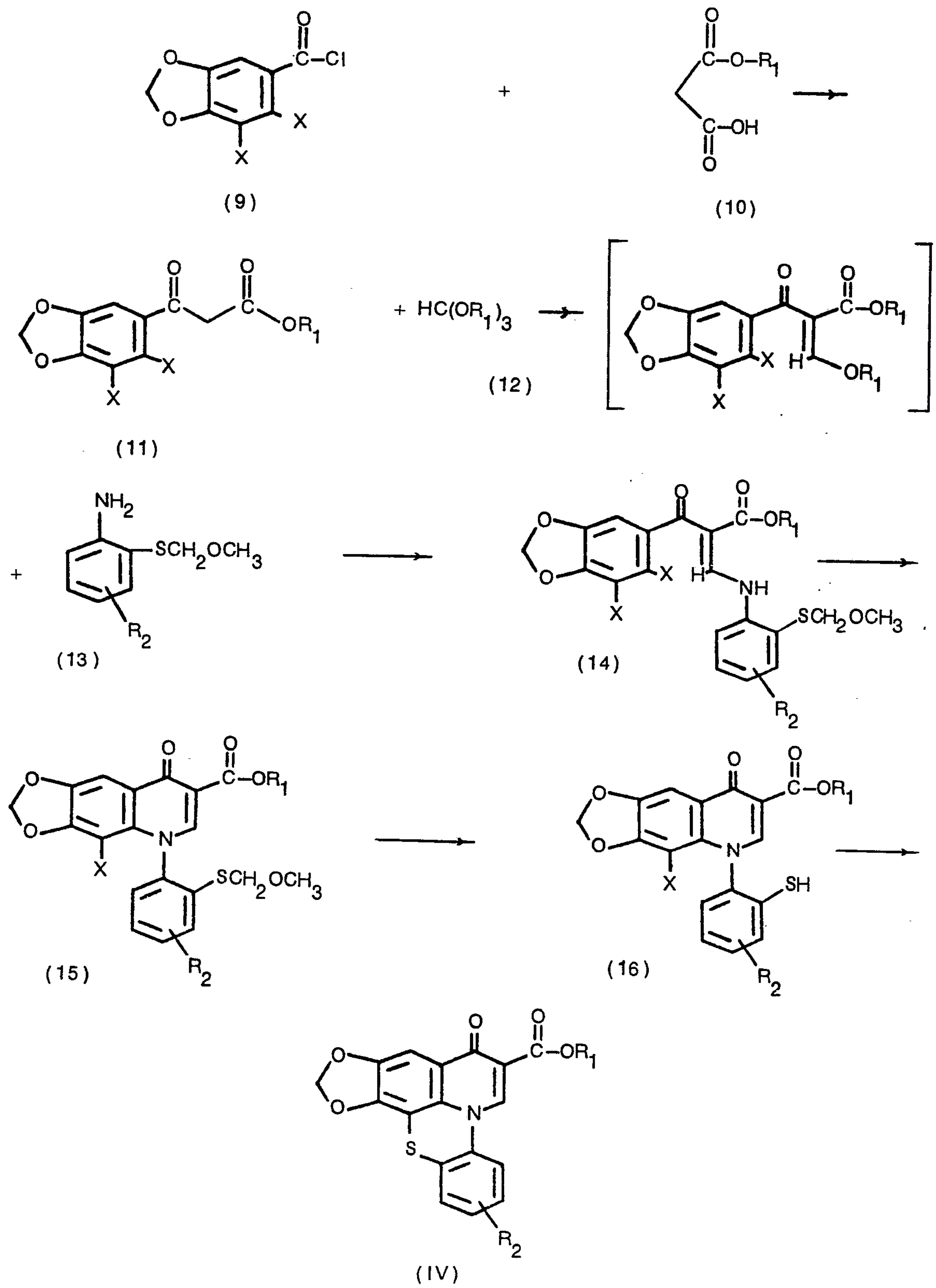

(9) (10) (11) (12) (13) (14) (15) (16) (IV)

In accordance with the foregoing reaction scheme, 2,3-dibromo-4,5-methylenedioxybenzoyl chloride is reacted with malonic acid halfester (10) in the presence of n-butyllithium to give the β-ketoester (11). The β-ketoester (11) is then treated with a trialkyl- orthoformate (12) in the presence of an acid anhydride,

followed by reaction with substituted 2-methyoxymethyl-thioaniline (13) to obtain the enaminoketoester (14). This enaminoketoester (14) is then cyclized by treatment with a strong base such as sodium hydride to obtain the 1-substituted-6,7-methylenedioxy-4-oxo-1,4-dihydroquinoline-3-carboxylic ester (15). The methoxymethylthioether moiety of (15) is removed by mineral strong acid or by borontrichloride to give the thiophenol (16). Cyclization of (16) with a strong base as defined above, preferably sodium hydride yields the ester (IV) which upon acid or base hydrolysis gives the free acid (IV), ($R_1$ = H).

As used in the following examples, the references to compounds such as (9), (10), (11), etc. and to the substituents, such as R, $R_1$, $R_2$ etc. refer to the corresponding compounds and substituents in the foregoing reaction scheme and in formula IV.

Example 21

1,2-methylenedioxy-4-oxo-4H-quino[2,3,4-i,j][1,4]benzothiazine-5-carboxylic acid

(a) to a dry ice cooled solution of 0.85 g. malonic acid monoethyl ester in 25 ml. of tetrahydrofuran (THF) is slowly added 9.2 ml. of 1.4 M n-butyl lithium in THF and the flask is warmed to −5°C. After 5 minutes, the solution is cooled back to −70°C. After 1.3 g. of the acid chloride (9) (X = Br) is added, the cooling bath is removed and warmed up to room temperature over an hour. The solution is then partitioned between 1N HCl and ether. The ether portion is washed with $NaHCO_3$ and is dried over $MgSO_4$, then evaporated to obtain a pale yellow oil which is then purified over a silica gel column eluted to yield β-ketoester (11) ($R_1$ = $C_2H_5$, X = Br).

(b) A solution of 1.25 g. of β-ketoester (11) ($R_1$ = $C_2H_5$, X = Br) in 0.8 ml. of triethylorthoformate and 5 ml. of acetic anhydride is heated at 135°C for 1-1/2 hours with the removal of the ethyl acetate formed during the reaction. The solution is evaporated under reduced pressure to a mobile oil. The oil is then dissolved in 5 ml. of methylene chloride and 0.45 g. of 2-methoxymethylthioaniline is added into the solution. After 1 hour, the solution is evaporated to dryness and crystallized from ethylacetate, yielding (14), wherein $R_1$ = $C_2H_5$, X = Br and $R_2$ = H.

(c) To a cold solution of 3.5 g. of the preceding product (14) in 170 ml. tetrahydrofuran (THF) is slowly added 0.20 g. of a 60% sodium hydride-in-oil suspension. The mixture is refluxed for 16 hours and is cooled and diluted with water to a volume of 500 ml. The mixture is then filtered to obtain (15) wherein $R_1$ = $C_2H_5$, X = Br and $R_2$ = H.

To a cold solution of the methoxymethylthio ether (15) (1 g.) in 20 ml. methylene chloride is added 2 ml. of boron trichloride with stirring. After 15 minutes at 0°C, the solution is evaporated to dryness under reduced pressure at room temperature yielding the crude thiophenol (16). This is then dissolved in 20 ml. O-dichlorobenzene. 1.35 mg. of sodium hydride is added and the mixture is heated at 135°C. for 24 hours. It is cooled and water containing dilute acetic acid is added and the mixture is extracted with methylene chloride (3 × 50 ml.). Purification of the crude product by chromatography yields (IV) ($R_1$ = $C_2H_5$, $R_2$ = H).

(d) To a suspension of 1.6 g. of (IV) ($R_1$ = $C_2H_5$, $R_2$ = H) in 20 ml. THF is added a sodium hydroxide solution (0.2 g. in 20 ml. water). The mixture is heated at 80°C. for 2 hours resulting in a clear solution which is evaporated under reduced pressure to dryness. The solid is dissolved in 200 ml. water and 1 ml. acetic acid is added. The resulting precipitate is filtered and washed with cold water to produce 1,2-methylenedioxy-4-oxo-4H-quino[2,3,4-i,j][1,4]benzothiazine-5-carboxylic acid (IV) ($R_1$ = H, $R_2$ = H).

Example 22

1,2-methylenedioxy-10-fluoro-4-oxo-4H-quino[2,3,4-i,j][1,4]benzothiazine-5-carboxylic acid

The procedure of Example 21 can be repeated replacing 2-methoxymethylthioaniline in Example 21(b) with 2-methoxymethylthio-4-fluoroaniline to obtain 1,2-methylenedioxy-10-fluoro-4-oxo-4H-quino[2,3,4-i,j][1,4]benzothiazine-5-carboxylic acid (IV) ($R_1$ = H, $R_2$ = 10-fluoro).

Example 23

In the described fashion as Example 21, replacing 2-methoxymethylthioaniline in Example 21(b) with appropriate substituted 2-methoxymethylthioaniline, one can obtain the additional substituted 1,2-methylenedioxy-4-oxo-4H-quino[2,3,4-i,j][1,4]-benzothiazine-5-carboxylic acid (IV) ($R_1$ = H) as listed in Table III.

TABLE III

| Substituted 2-methoxy-methylthioaniline | Compound IV obtained ($R_1$ = H) |
|---|---|
| 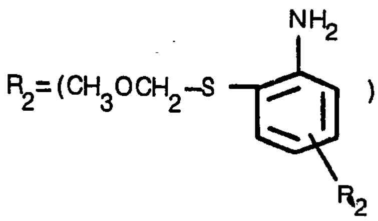 | $R_2$ = |
| (a) 6-fluoro | 8-fluoro |
| (b) 5-fluoro | 9-fluoro |
| (c) 3-fluoro | 11-fluoro |
| (d) 4,6-difluoro | 8,10-difluoro |
| (e) 4-chloro | 10-fluoro |
| (f) 4-methyl | 10-methyl |
| (g) 4,5-methylenedioxy | 9,10-methylenedioxy |
| (h) 4-hydroxy | 10-hydroxy |
| (i) 4-methoxy | 10-methoxy |

The compounds of Formula V wherein Z is a substituted or unsubstituted pyridine ring may be prepared in accordance with the following reaction scheme:

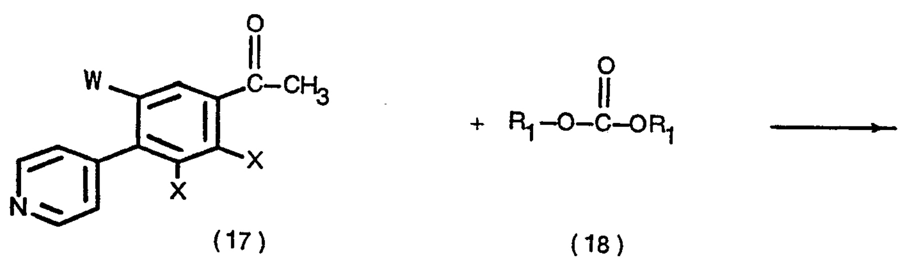

(17) (18)

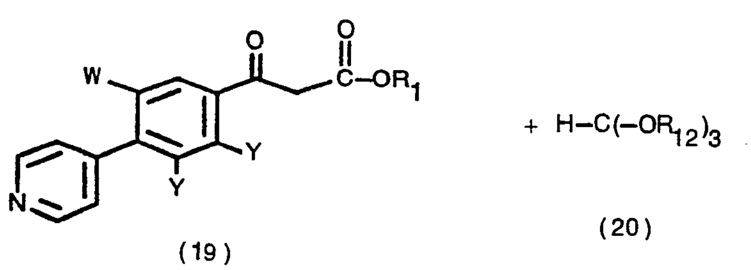

(19) (20)

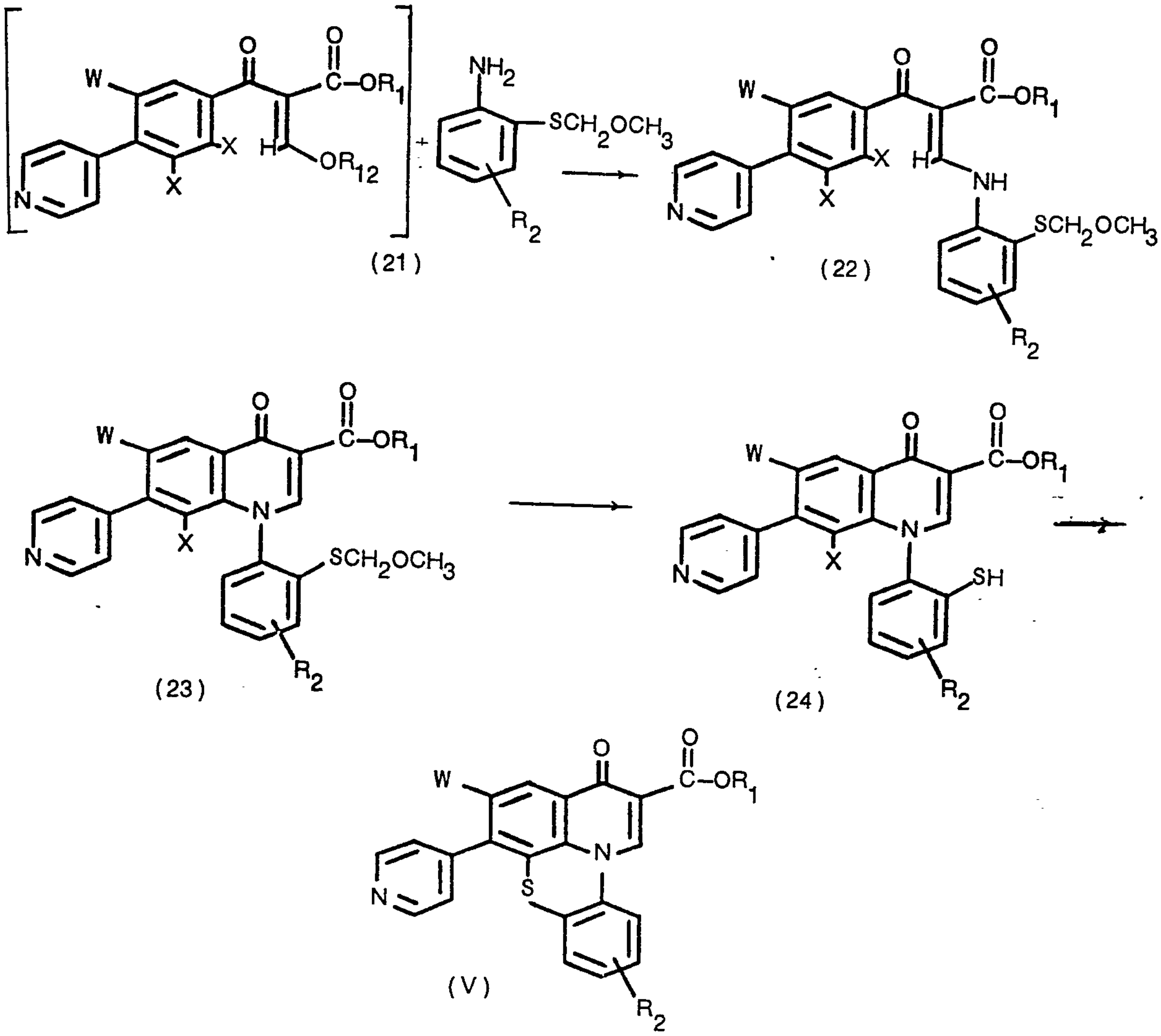

(21) (22) (23) (24) (V)

In accordance with the foregoing reaction scheme wherein X is a halogen or a leaving group, the acetophenone (17) is reacted with a dialkoxycarbonate (18) in the presence of a strong base to obtain the corresponding β-ketoester (19). In the dialkoxycarbonate (18), $R_1$ may be an alkyl group of, for example, 1 to 10 carbon atoms, but is preferably loweralkyl, such as ethyl. Suitable bases include metal hydrides, such as sodium hydride, potassium hydride and the like, as well as metal alkoxides in alcohol, such as sodium ethoxide in ethanol. The preferred base is sodium hydride. Formation of the β-ketoester (19) is facilitated by reacting the acetophenone (17) with the dialkoxycarbonate (18) at elevated temperatures, such as from about 20°C to about 120°C, and preferably from about 30°C to about 90°C until completion of the reaction. The β-ketoester may then be separated from the reaction mixture in a conventional manner.

The β-ketoester (19) is then treated with a trialkylorthoformate (20) in the presence of an acid anhydride, preferably acetic anhydride, followed by reaction with substituted or unsubstituted 2-methoxymethylthioaniline to obtain the enamino-ketoester (22). In the trialkylorthoformate (20), $R_{12}$ may be an alkyl group of, for example, from 1 to 10 carbon atoms, but is preferably loweralkyl, such as ethyl. Reaction with the trialkylorthoformate is preferably conducted at elevated temperatures, such as from about 50°C to about 150°C, and preferably from about 100°C to about 140°C, to obtain an oily liquid, which may be isolated or unisolated, as desired (shown in brackets in the reaction scheme). Reaction of the latter with the substituted or unsubstituted 2-methoxymethylthioaniline (21) is preferably conducted in an appropriate aprotic or non-aprotic solvent, preferably methylene chloride or tetrahydrofuran, and may be conducted at room or suitable elevated temperatures, as desired.

The enamino-ketoester (22) is then cyclized, such as by treatment with a strong base as defined above, preferably sodium hydride, to obtain the 1,4-dihydro-4-oxo-quinoline-3-carboxylic acid ester (23). Cyclization is conducted in the presence of an aprotic solvent, such as dimethoxyethane, dimethylformamide, tetrahydrofuran or chlorobenzene, and is preferably conducted at temperatures of about 20°C, to about 145°C, and more preferably at the reflux temperature of the solvent employed.

The methoxymethylthio ether moiety of (23) is then removed by mineral strong acid or by borontrichloride to give the thiophenol (24). Cyclization of (24) with a strong base as defined above,

preferably sodium hydride, yields the ester (V) ($R_1$ = alkyl) which upon acid or base hydrolysis gives the free acid (V) ($R_1$ = H).

The 4-oxo-4H-quino[2,3,4-i,j][1,4]benzothiazine-5-carboxylic acid (V) can be converted into the corresponding ester, if desired, by conventional esterification procedures, such as by treating the free acid (V) with the appropriate alcohol in the presence of an acid catalyst, by converting the free acid (V) into the corresponding acid chloride followed by displacement of the chloro radical with the appropriate alcohol, or by treating the sodium salt of the acid (V) with a suitable reactive halide, such as chloromethylpivalate or dimethylaminoethylchloride in dimethoxyethane to obtain, for example, the pivaloyloxymethyl ester or dimethylaminoethylester (V).

The foregoing may be better understood from the following examples, which are presented for purposes of illustration and are not intended to limit the scope of the inventive concepts. As used in the following examples, the references to compounds, such as (17), (18), (19), etc., and to substituents, such as R, $R_1$, $R_2$, etc., refer to the corresponding compounds and substituents in the foregoing reaction scheme and in formula V.

Example 24

1-(4-pyridyl)-2-fluoro-4-oxo-4H-quino[2,3,4-i,j][1,4]benzothiazine-5-carboxylic acid

(a) A cold solution of 28.4 g. 2,3-dichloro-4-(4-pyridyl)-5-fluoroacetophenone in 350 ml. diethylcarbonate is slowly added to 8.0 g. 60% sodium hydride-in-oil suspension. The mixture is heated at 80°C for 3 hours, then poured into 700 ml. ice cold water solution containing 25 ml. acetic acid. The mixture is extracted with three 400 ml. portions of ether. The organic phase is dried over $MgSO_4$, evaporated and the obtained oil is purified through silica gel column to give pure (19). (X = Cl, $R_1$ = $C_2H_5$, W = F).

(b) A solution of 16.6 g. of β-ketoester (19) ($R_1$ = $C_2H_5$, X = Cl, W = F) in 14 ml. of triethylorthoformate and 35 ml. of acetic anhydride is heated at 135°C for 1-1/2 hours with the removal of the ethyl acetate formed during the reaction. The solution is evaporated under reduced pressure to a mobile oil. The oil is then dissolved in 150 ml. of methylene chloride and 7.5 g. of 2-methoxymethylthioaniline is added into the solution. After 1 hour, the solution is evaporated to dryness yielding (22), wherein $R_1$ = $C_2H_5$, X = Cl, $R_2$ = H, W = F.

(c) To a cold solution of 14 g. of the preceding product (22) ($R_1$ = $C_2H_5$, $R_2$ = H, X = Cl, W = F), in 140 ml. tetrahydrofuran (THF) is slowly added 1.25 g. of a 60% sodium hydride-in-oil suspension. The mixture is refluxed for 6 hours and is cooled and diluted with water to a volume of 1.5 liters. The mixture is then filtered and the solid is washed with 1:1 hexane/ether solution to obtain (23) wherein $R_1$ = $C_2H_5$, W = F and $R_2$ = H).

To a solution of the methoxymethylthio ether (23) 2 g. in 30 ml. methylenechloride is added 2.5 ml. of boron trichloride with stirring. After 15 minutes at 0°C, the solution is evaporated under reduced pressure at room temperature yielding the crude thiophenol (24). This is then dissolved in 20 ml. O-dichlorobenzene. 185 mg. of sodium hydride is added and the mixture is heated at 135°C for 24 hours. It is cooled and water containing dilute acetic acid is added and the mixture is extracted with methylene chloride (3 × 100 ml.). Purification of the crude product by chromatography yields (V) ($R_1$ = $C_2H_5$, $R_2$ = H, W = F).

(d) To a suspension of 2 g. of (V) ($R_1$ = $C_2H_5$, $R_2$ = H, W = F) in 30 ml. THF is added a sodium hydroxide solution (0.25 g. in 5 ml. water). The mixture is heated at 80°C for 3 hours resulting in a clear solution which is evaporated to dryness. The residue is dissolved in 100 ml. water and 2 ml. acetic acid is added. The resulting precipitate is filtered and washed with cold water to produce 1-(4-pyridyl)-2-fluoro-4-oxo-4H-quino[2,3,4-i,j][1,4]benzothiazine-5-carboxylic acid (V), ($R_1$ = $R_2$ = H, W = F).

Example 25

1-(4-pyridyl)-2,10-difluoro-4-oxo-4H-quino[2,3,4-i,j][1,4]benzothiazine-5-carboxylic acid

The procedure of Example 24 can be repeated replacing 2-methoxymethylthioaniline with 2-methoxymethylthio-4-fluoro-aniline in Example 24(b) to obtain 1-(4-pyridyl)-2,10-difluoro-4-oxo-4H-quino[2,3,4-i,j]-[1,4]benzothiazine-5-carboxylic acid (V) ($R_1$ = H, $R_2$ = 10-fluoro, W = F).

Example 26

In the described fashion as Example 24 replacing 2-methoxymethylthio aniline in Example 24(b) with 2-methoxymethylthio-4-hydroxy-aniline, one can obtain 1-(4-pyridyl)-2-fluoro-10-hydroxy-4-oxo-4H-quino[2,3,4-i,j][1,4]benzothiazine-5-carboxylic acid (V) ($R_1$ = H, $R_2$ = 10-hydroxy, W = F).

Example 27

The procedure of Example 24 can be repeated replacing 2-methoxymethylthio-aniline in Example 24(b) with 2-methoxymethylthio-4-methoxyaniline to obtain 1-(4-pyridyl)-2-fluoro-10-methoxy-4-oxo-4H-quino[2,3,4-i,j][1,4]benzothiazine-5-carboxylic acid (V), ($R_1$ = H, $R_2$ = 10-methoxy, W = F).

Example 28

In the described fashion as Example 24 replacing 2-methoxymethylthio-aniline in Example 24(b) with various substituted 2-methoxymethyl thio aniline, one can obtain additional substituted 1-(4-pyridyl)-2-fluoro-4-oxo-4H-quino[2,3,4-i,j][1,4]benzothiazine-5-carboxylic acid (V) as summarized in Table IV.

TABLE IV

| 2-Methoxymethylthio-aniline Replacement | Compound V obtained ($W = F$, $R_1 = H$) |
|---|---|
| 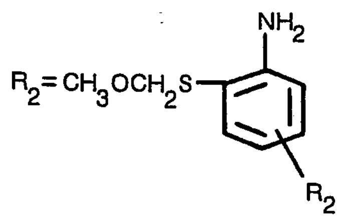 | $R_2$ = |
| (a) 6-fluoro | 8-fluoro |
| (b) 5-fluoro | 9-fluoro |
| (c) 3-fluoro | 11-fluoro |
| (d) 4,6-difluoro | 8,10-difluoro |
| (e) 4-chloro | 10-fluoro |
| (f) 4-methyl | 10-methyl |
| (g) 4,5-methylenedioxy | 9,10-methylenedioxy |

Example 29

In the described fashion as Example 24 replacing 2,3-dichloro-4-(4-pyridyl)-5-fluoroacetophenone in Example 24(a) with 2,3-dichloro-4-(4-pyridyl)acetophenone and optionally 2-methoxymethylthio-aniline in Example 24(b) with 2-methoxymethylthio-fluoroaniline or 2-methoxymethylthio-4,5-methylenedioxy-aniline, one can obtain the following three compounds:

(a) 1-(4-pyridyl)-4-oxo-4H-quino[2,3,4-i,j][1,4]benzothiazine-5-carboxylic acid (V), ($R_1$ = H, W = H, $R_2$ = H).

(b) 1-(4-pyridyl)-10-fluoro-4-oxo-4H-quino[2,3,4-i,j][1,4]benzothiazine-5-carboxylic acid (V) ($R_1$ = H, W = H, $R_2$ = 10-fluoro).

(c) 1-(4-pyridyl)-9,10-methylenedioxy-4-oxo-4H-quino[2,3,4-i,j][1,4]benzothiazine-5-carboxylic acid (V) ($R_1$ = H, W = H, $R_2$ = 9,10-methylenedioxy).

**Claims**

1. A compound having the formula

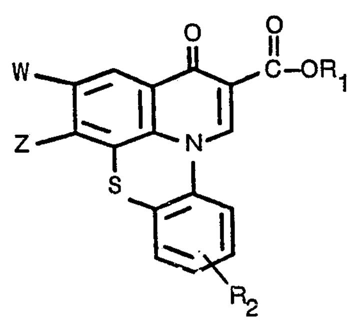

wherein $R_1$ is hydrogen or a carboxy protecting group; $R_2$ is one or more groups selected from the group consisting of hydrogen, halogen, methylenedioxy, $C_1$ to $C_6$ alkyl and substituted derivatives thereof, wherein said substituents are selected from hydroxy and halo groups, a group having the formula:

$$—O—R_3$$

wherein $R_3$ is hydrogen or $C_1$ to $C_6$ alkyl, and an amine group having the formula:

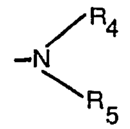

wherein $R_4$ and $R_5$ are independently hydrogen or $C_1$ to $C_6$ alkyl; W is halogen or hydrogen; and Z is selected from the group consisting of an aliphatic heterocyclic ring having the formula

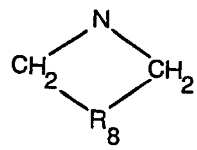

wherein $R_8$ is methylene, dimethylene or a group of the formula $-CH_2-CH_2-R_9-CH_2-$ or $-CH_2-R_9-CH_2-$ wherein $R_9$ is selected from the group consisting of —S—, —O— and —N—; substituted derivatives thereof wherein said substituents are selected from the group consisting of $C_1$ to $C_6$ alkyl, $C_1$ to $C_6$ hydroxyalkyl, hydroxy, alkanoyl containing 1 to 6 carbon atoms, alkanoylamido containing 1 to 6 carbon atoms and an amine of the formula

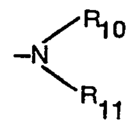

wherein $R_{10}$ and $R_{11}$ are each independently selected from the group consisting of hydrogen, $C_1$ to $C_6$ alkyl and substituted derivatives thereof, wherein said substituents are selected from hydroxy and halo groups; an amino group of the formula:

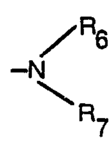

wherein $R_6$ is hydrogen or $C_1$ to $C_{10}$ alkyl and $R_7$ is selected from the group consisting of $C_1$ to $C_{10}$ alkyl and hydroxy substituted derivatives thereof, an amine group, a mono-($C_1$ to $C_6$) alkylamino group and a di-($C_1$ to $C_6$) alkylamino group; a pyridine ring of the formula

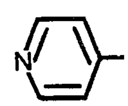

or substituted derivatives of the pyridine ring wherein said substituents are selected from the group consisting of $C_1$ to $C_6$ alkyl, halogen, a group of the formula $-Y-R_{12}$ wherein Y is —O— or —S— and $R_{12}$ is lower alkyl, hydroxy, alkanoyl containing 1 to 6 carbon atoms, alkanoylamido containing 1 to 6 carbon atoms and an amine of the formula:

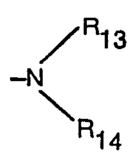

wherein $R_{13}$ and $R_{14}$ are each independently selected from the group consisting of hydrogen, $C_1$ to $C_6$ alkyl and substituted derivatives thereof, wherein said substituents are selected from hydroxy and halo groups; or, alternatively, W and Z together may form a methylenedioxy bridge, and pharmaceutically acceptable salts thereof.

2. A compound as defined in Claim 1 wherein W is F; Z is piperazinyl, $C_1$ to $C_6$ alkyl-substituted piperazinyl, 4-acylpiperazinyl, or 3-amino or substituted aminopyrrolidinyl wherein said substituents on the

amino group are selected from $C_1$ to $C_6$ alkyl and hydroxy- or halo-substituted $C_1$ to $C_6$ alkyl, and $R_2$ is one or more of a $C_1$ to $C_6$ alkyl group, a hydrogen atom, a halogen atom, a hydroxy group and a methylenedioxy group, and pharmaceutically acceptable salts thereof.

3. A compound as defined in Claim 2 wherein $R_2$ is hydrogen; Z is piperazinyl or 4-methylpiperazinyl; and $R_1$ is hydrogen.

4. A compound as defined in Claim 2 wherein $R_2$ is hydrogen, 10-hydroxy, 10-fluoro or 8,10 difluoro; Z is piperazinyl, 4-methylpiperazinyl, 3-aminopyrrolidinyl, 3-methylaminopyrrolidinyl or dimethylamino-pyrrolidinyl; and $R_1$ is hydrogen.

5. A compound as defined in Claim 1 wherein W and Z together form a methylenedioxy bridge; $R_1$ is hydrogen or a carboxy protecting group; $R_2$ is one or more groups selected from the group consisting of hydrogen, halogen, methylenedioxy, $C_1$ to $C_6$ alkyl and substituted derivatives thereof, wherein said substituents are selected from hydroxy and halo groups, a group having the formula:

$$—O—R_3$$

wherein $R_3$ is hydrogen or $C_1$ to $C_6$ alkyl, and an amine group having the formula:

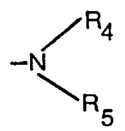

wherein $R_4$ and $R_5$ are independently hydrogen or $C_1$ to $C_6$ alkyl; and, pharmaceutically acceptable salts thereof.

6. A compound as defined in Claim 5 wherein $R_2$ is hydrogen, $C_1$ to $C_6$ alkyl, 10-fluoro, methylenedioxy, or 8,10-difluoro group; and $R_1$ is hydrogen.

7. A compound as defined in Claim 1 wherein $R_1$ is hydrogen or a carboxy protecting group; W is halogen or hydrogen; $R_2$ is one or more groups selected from the group consisting of hydrogen, halogen, methylenedioxy, $C_1$ to $C_6$ alkyl and substituted derivatives thereof, wherein said substituents are selected from hydroxy and halo groups, a group having the formula:

$$—O—R_3$$

wherein $R_3$ is hydrogen or $C_1$ to $C_6$ alkyl, and an amine group having the formula:

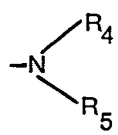

wherein $R_4$ and $R_5$ are independently hydrogen or $C_1$ to $C_6$ alkyl; and Z is a pyridine ring and pharmaceutically acceptable salts thereof.

8. A compound as defined in Claim 7 wherein $R_2$ is hydrogen, hydroxy, fluoro, difluoro or methylenedioxy; $R_1$ is hydrogen, and W is fluoro or hydrogen.

**Patentansprüche**

1. Verbindung mit der Formel

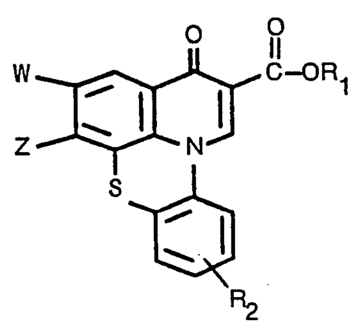

worin $R_1$ Wasserstoff oder eine Carboxyschutzgruppe ist; $R_2$ eine oder mehrere Gruppen ist, die aus der

aus Wasserstoff, Halogen, Methylendioxy, $C_1$- bis $C_6$-Alkyl und substituierten Derivaten davon, worin die Substituenten aus Hydroxy- und Halogengruppen ausgewählt sind, einer Gruppe mit der Formel

$$—O—R_3,$$

worin $R_3$ Wasserstoff oder $C_1$- bis $C_6$-Alkyl ist, und einer Aminogruppe mit der Formel

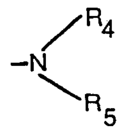

worin $R_4$ und $R_5$ unabhängig Wasser oder $C_1$- bis $C_6$-Alkyl sind, bestehenden Gruppe ausgewählt sind; W Halogen oder Wasserstoff ist; und Z aus der Gruppe ausgewählt ist, bestehend aus einem aliphatischen, heterocyclischen Ring mit der Formel

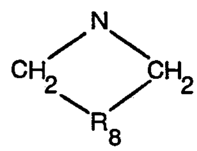

worin $R_8$ Methylen, Dimethylen oder eine Gruppe der Formel $—CH_2—CH_2—R_9—CH_2—$ oder $—CH_2—R_9—CH_2—$ ist, worin $R_9$ aus der aus —S—, —O— und —N— bestehenden Gruppe ausgewählt ist; substituierten Derivaten davon, worin die Substituenten aus der aus $C_1$- bis $C_6$-Alkyl, $C_1$- bis $C_6$-Hydroxyalkyl, Hydroxy, Alkanoyl mit 1 bis 6 Kohlenstoffatomen, Alkanoylamido mit 1 bis 6 Kohlenstoffatomen und einem Amin der Formel

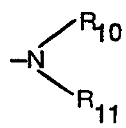

worin $R_{10}$ und $R_{11}$ unabhängig jeweils aus der aus $C_1$- bis $C_6$-Alkyl und substituierten Derivaten davon bestehenden Gruppe ausgewählt sind, worin die Substituenten aus Hydroxy- und Halogengruppen ausgewählt sind; einer Aminogruppe der Formel

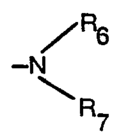

worin $R_6$ Wasserstoff oder $C_1$- bis $C_{10}$-Alkyl ist und $R_7$ aus der aus $C_1$- bis $C_{10}$-Alkyl und hydroxysubstituierten Derivaten davon, einer Aminogruppe, einer Mono($C_1$ bis $C_6$)alkylaminogruppe und einer Di($C_1$ bis $C_6$)alkylaminogruppe bestehenden Gruppe ausgewählt ist; einem Pyridinring der Formel

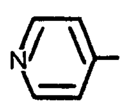

oder substituierten Derivaten des Pyridinrings, worin die Substituenten aus der Gruppe ausgewählt sind, welche aus $C_1$- bis $C_6$-Alkyl, Halogen, einer Gruppe der Formel $—Y—R_{12}$ besteht, worin Y —O— oder —S— ist und $R_{12}$ Niederalkyl, Hydroxy, Alkanoyl mit 1 bis 6 Kohlenstoffatomen, Alkanoylamino mit 1 bis 6 Kohlenstoffatomen und ein Amin der Formel

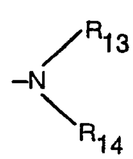

worin $R_{13}$ und $R_{14}$ jeweils unabhängig aus der aus Wasserstoff, $C_1$- bis $C_6$-Alkyl und substituierten Derivaten davon bestehenden Gruppe ausgewählt sind, worin die Substituenten aus Hydroxy- und Halogengruppe ausgewählt sind, ist, besteht; oder, alternativ, W und Z zusammengenommen eine Methylendioxybrücke bilden können, sowie pharmazeutisch annehmbare Salze davon.

2. Verbindung, wie in Anspruch 1 definiert, worin W F ist; Z Piperazinyl, $C_1$- bis $C_6$-Alkyl-substituiertes Piperazinyl, 4-Acylpiperazinyl oder 3-Amino- oder substituiertes Aminopyrrolidinyl, worin die Substituenten an der Aminogruppe aus $C_1$- bis $C_6$-Alkyl und Hydroxy- oder Halogen-substituiertem $C_1$- bis $C_6$-Alkyl ausgewählt sind, ist und $R_2$ eines oder mehrere aus einer $C_1$- bis $C_6$-Alkylgruppe, einem Wasserstoffatom, einem Halogenatom, einer Hydroxygruppe und einer Methylendioxygruppe ist, sowie pharmazeutisch annehmbare Salze davon.

3. Verbindung, wie in Anspruch 2 definiert, worin $R_2$ Wasserstoff ist, Z Piperazinyl oder 4-Methylpiperazinyl ist und $R_1$ Wasserstoff ist.

4. Verbindung, wie in Anspruch 2 definiert, worin $R_2$ Wasserstoff, 10-Hydroxy, 10-Fluor oder 8,10-Difluor ist; Z Piperazinyl, 4-Methylpiperazinyl, 3-Aminopyrrolidinyl, 3-Methylaminopyrrolidinyl oder Dimethylaminopyrrolidinyl ist und $R_1$ Wasserstoff ist.

5. Verbindung, wie in Anspruch 1 definiert, worin W und Z zusammen eine Methylendioxybrücke bilden, $R_1$ Wasserstoff oder eine Carboxyschutzgruppe ist, $R_2$ eine oder mehrere Gruppen ist, die aus der aus Wasserstoff, Halogen, Methylendioxy, $C_1$- bis $C_6$-Alkyl und substituierten Derivaten davon, worin die Substituenten aus Hydroxy- und Halogengruppen ausgewählt sind, einer Gruppe mit der Formel

$$-O-R_3,$$

worin $R_3$ Wasserstoff oder $C_1$- bis $C_6$-Alkyl ist, und einer Aminogruppe mit der Formel

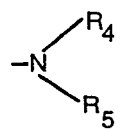

worin $R_4$ und $R_5$ unabhängig Wasserstoff oder $C_1$- bis $C_6$-Alkyl sind, bestehenden Gruppe ausgewählt sind, sowie pharmazeutisch annehmbare Salze davon.

6. Verbindung, wie in Anspruch 5 definiert, worin $R_2$ Wasserstoff, $C_1$- bis $C_6$-Alkyl, 10-Fluor, Methylendioxy, eine 8,10-Difluorgruppe ist und $R_1$ Wasserstoff ist.

7. Verbindung, wie in Anspruch 1 definiert, worin $R_1$ Wasserstoff oder eine Carboxyschutzgruppe ist, W Halogen oder Wasserstoff ist, $R_2$ eine oder mehrere Gruppen ist, die aus der aus Wasserstoff, Halogen, Methylendioxy, $C_1$- bis $C_6$-Alkyl und substituierten Derivaten davon, worin die Substituenten aus Hydroxy- und Halogengruppen ausgewählt sind, einer Gruppe mit der Formel

$$-O-R_3,$$

worin $R_3$ Wasserstoff oder $C_1$- bis $C_6$-Alkyl ist, und eine Aminogruppe mit der Formel

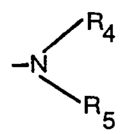

worin $R_4$ und $R_5$ unabhängig Wasserstoff oder $C_1$- bis $C_6$-Alkyl sind, bestehenden Gruppe ausgewählt sind, und Z ein Pyridinring ist, sowie pharmazeutisch annehmbare Salze davon.

8. Verbindung, wie in Anspruch 7 definiert, worin $R_2$ Wasserstoff, Hydroxy, Fluor, Difluor oder Methylendioxy ist, $R_1$ Wasserstoff ist und W Fluor oder Wasserstoff ist.

**Revendications**

1. Composé répondant à la formule

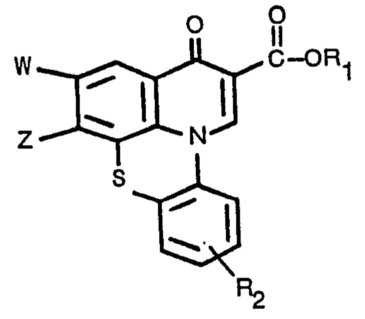

dans lequel $R_1$ représente un atome d'hydrogène ou un groupe protecteur de fonction carboxyle; $R_2$ représente un ou plusieurs groupes choisis parmi un atome d'hydrogène, un atome d'halogène, un groupe méthylènedioxy, un groupe alkyle en $C_1$ à $C_6$ et ses dérivés de substitution, dans lesquels lesdits substituants sont choisis parmi des groupes hydroxy halogénaux, un groupe de formule:

$$—O—R_3$$

dans laquelle $R_3$ représente un atome d'hydrogène ou un groupe alkyle en $C_1$ à $C_6$, et un groupe amine répondant à la formule:

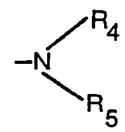

dans laquelle $R_4$ et $R_5$ représentent, indépendamment, un atome d'hydrogène ou un groupe alkyle en $C_1$ à $C_6$; W représente un atome d'halogène ou d'hydrogène; et Z est choisi dans l'ensemble consistant en un noyau hétérocyclique aliphatique de formule:

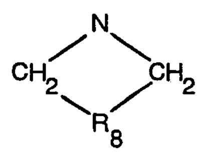

dans laquelle $R_8$ représente un groupe méthylène, diméthylène ou un groupe de formule $—CH_2—CH_2—R_9—CH_2—$ ou $—CH_2—R_9—CH_2—$, où $R_9$ est choisi parmi —S—, —O—, —N—; leurs dérivés de substitution, dans lesquels lesdits substituants sont choisis parmi un groupe alkyle en $C_1$ à $C_6$, un groupe hydroxyalkyle en $C_1$ à $C_6$, un groupe hydroxyle, un groupe alcanoyle contenant 1 à 6 atomes de carbone, un groupe alcanoylamido contenant 1 à 6 atomes de carbone et une amine de formule

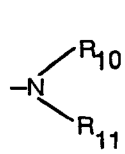

dans laquelle $R_{10}$ et $R_{11}$ sont choisis chacun, indépendamment, parmi un atome d'hydrogène, un groupe alkyle en $C_1$ à $C_6$ et ses dérivés de substitution, lesdits substituants étant choisis parmi un groupe hydroxyle et un groupe halogéno; un groupe amino de formule

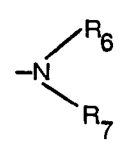

dans laquelle $R_6$ représente un atome d'hydrogène ou un groupe alkyle en $C_1$ à $C_{10}$ et $R_7$ est choisi parmi un

groupe allyle en $C_1$ à $C_{10}$ et ses dérivés de substitution, un groupe amine, un groupe mono-alkyl (en $C_1$ à $C_6$) amino et un groupe di-alkyl (en $C_1$ à $C_6$) amino; un noyau pyridine de formule

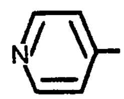

ou des dérivés de substitution du noyau pyridine, dans lesquels lesdits substituants sont choisis parmi un groupe alkyle en $C_1$ à $C_6$, halogéno, un groupe de formule —Y—$R_{12}$ dans laquelle Y représente —O— ou —S— et $R_{12}$ représente un groupe alkyle inférieur, hydroxyle, alcanoyle contenant 1 à 6 atomes de carbone, alcanoylamido contenant 1 à 6 atomes de carbone et une amine de formule:

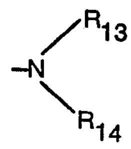

dans laquelle $R_{13}$ et $R_{14}$ sont choisis chacun, indépendamment, parmi un atome d'hydrogène, un groupe alkyle en $C_1$ à $C_6$ et ses dérivés de substitution, lesdits substituants étant choisis parmi les groupes hydroxyles et halogéno; ou bien W et Z peuvent former ensemble un pont méthylènedioxy, et leurs sels pharmaceutiquement acceptables.

2. Composé tel que défini à la revendication 1, dans lequel W représente F; Z représente un groupe pipérazinyle, pipérazinyle substitué par un groupe alkyle en $C_1$ à $C_6$, 4-acylpipérazinyle ou 3-aminopyrrolidinyle ou 3-(amino-substitué) pyrrolidinyle, dans lequel les substituants fixés sur le groupe amino sont choisis parmi un groupe alkyle en $C_1$ à $C_6$ et un groupe alkyle en $C_1$ à $C_6$ (substitué par un groupe hydroxyle ou par de l'halogène), et $R_2$ représente un ou plusieurs des groupes suivants: un groupe alkyle en $C_1$ à $C_6$, un atome d'hydrogène, un atome d'halogène, un groupe hydroxyle et un groupe méthylènedioxy, et ses sels pharmaceutiquement acceptables.

3. Composé tel que défini à la revendication 2, dans lequel $R_2$ représente un atome d'hydrogène; Z représente un groupe pipérazinyle ou 4-méthylpipérazinyle; et $R_1$ est un atome d'hydrogène.

4. Composé tel que défini à la revendication 2, dans lequel $R_2$ représente un atome d'hydrogène, un groupe 10-hydroxy, 10-fluoro ou 8,10-difluoro; Z est un groupe pipérazinyle, 4-méthylpipérazinyle, 3-aminopyrrolidinyle, 3-méthylaminopyrrolidinyle ou diméthylaminopyrrolidinyle; et $R_1$ représente un atome d'hydrogène.

5. Composé tel que défini à la revendication 1, dans lequel W et Z forment ensemble un pont méthylènedioxy; $R_1$ représente un atome d'hydrogène ou un groupe protecteur de fonction carboxyle; $R_2$ représente un ou plusieurs groupes choisis parmi un atome d'hydrogène, un atome d'halogène, un groupe méthylènedioxy, un groupe alkyle en $C_1$ à $C_6$ et ses dérivés de substitution, lesdits dérivés étant choisis parmi un groupe hydroxyle et un groupe halogéno; un groupe de formule:

$$—O—R_3$$

dans laquelle $R_3$ représente un atome d'hydrogène ou un groupe alkyle en $C_1$ à $C_6$, et un groupe amine de formule:

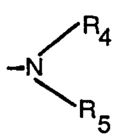

dans laquelle $R_4$ et $R_5$ représentent, indépendamment, chacun un atome d'hydrogène ou un groupe alkyle en $C_1$ à $C_6$; et ses sels pharmaceutiquement acceptables.

6. Composé tel que défini à la revendication 5, dans lequel $R_2$ représente un atome d'hydrogène, un groupe alkyle en $C_1$ à $C_6$, un groupe 10-fluoro, un groupe méthylènedioxy ou un groupe 8,10-difluoro; et $R_1$ représente un atome d'hydrogène.

7. Composé tel que défini à la revendication 1, dans lequel $R_1$ représente un atome d'hydrogène ou un groupe protecteur de fonction carboxyle; W représente un atome d'halogène ou d'hydrogène; $R_2$ représente un ou plusieurs groupes choisis parmi un atome d'hydrogène, un atome d'halogène, un groupe méthylènedioxy, alkyle en $C_1$ à $C_6$ et ses dérivés de substitution, lesdits substituants étant choisis parmi les groupes hydroxyles et halogéno; un groupe de formule:

$—O—R_3$

dans laquelle $R_3$ représente un atome d'hydrogène ou un groupe alkyle en $C_1$ à $C_6$, et un groupe amino de formule:

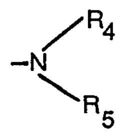

dans laquelle $R_4$ et $R_5$ représentent chacun, indépendamment, un atome d'hydrogène ou un groupe alkyle en $C_1$ à $C_6$; et Z représente un noyau pyridine, et ses sels pharmaceutiquement acceptables.

8. Composé tel que défini à la revendication 7, dans lequel $R_2$ représente un atome d'hydrogène, un groupe hydroxyle, fluoro, difluoro ou méthylènedioxy; $R_1$ représente un atome d'hydrogène et W représente un atome de fluor ou d'hydrogène.